# EUROPEAN PATENT APPLICATION

(11) **EP 3 788 943 A1**
(43) Date of publication of application: **10.03.2021**
(21) Application number: 19196000.4
(22) Date of filing: 06.09.2019
(51) Int. Cl.: A61B 1/00, A61B 1/04, A61B 1/05, G02B 23/24, H04N 5/225, H05K 3/32, H05K 3/34

(54) **A TIP PART ASSEMBLY FOR AN ENDOSCOPE**

(71) Applicant: Ambu A/S, 2750 Ballerup (DK)
(72) Inventor: OHLSSON, Jonas Mathias, 261 61 Landskrona (SE); OLSSON, Linus Rickard Frantzich, 237 31 Bjärred (SE); SØRENSEN, Morten, 2750 Ballerup (DK)
(74) Representative: AWA Denmark A/S

(57) **Abstract**

The present disclosure relates to a tip part assembly for an endoscope, comprising:
a circuit board comprising a first major surface and a second major surface opposite the first major surface, wherein a first electrical connection pad is positioned on the first major surface, and a second electrical connection pad is positioned on the second major surface, and
a camera module comprising a connection surface with at least a first and a second connection point for electrical communication of the camera module with the circuit board, the first and second connection points being spaced apart from each other,
wherein a solidified electrical conductor filler material directly electrically connects the first connection point of the connection surface and the first electrical connection pad of the circuit board, and a solidified electrical conductor filler material directly electrically connects the second connection point of the connection surface and the second electrical connection pad of the circuit board.

## Description

### Technical field

The present disclosure relates to endoscopes and more specifically to a tip part assembly for an endoscope.

### Background

Endoscopes are well known for visually inspecting places that are difficult to access, such as human body cavities. Typically, the endoscope comprises an elongated insertion tube with a handle at the proximal end, as seen from the operator, and visual inspection means, such as a built-in camera, at the distal end of the elongated insertion tube. This definition of the terms distal and proximal, i.e. proximal being the end closest to the operator and distal being the end remote from the operator, as used herein for endoscopes in general, is adhered to in the present specification.

As the name indicates, endoscopes are used for seeing inside things, such as lungs or other human body cavities of a patient. Modern endoscopes are therefore typically equipped with a light source and a vision receptor including a vision sensor, such as a camera or an image sensor. Provided that sufficient light is present, it is possible for the operator to see where the endoscope is steered and to set the target of interest once the tip has been advanced thereto. This therefore normally requires illumination of the area in front of the distal tip of the endoscope, in particular the field of vision of the camera(s). The light source, such as a light emitting diode, LED, or an optical fibre, may provide illumination.

Electrical wiring for the camera and other electronics, such as LED lighting accommodated in the tip part assembly at the distal end, run along the inside of the elongated insertion tube from the handle to the tip part assembly. Instead of using cameras, endoscopes may also be fibre-optic, in which case the optical fibres run along the inside of the elongated insertion tube to the tip part assembly. For some applications, a working or suction channel may run along the inside of the insertion tube from the handle to the tip part assembly, e.g. allowing liquid to be removed from the body cavity or allowing for insertion of surgical instruments or the like, into the body cavity. The suction channel may be connected to a suction connector, typically positioned at a handle at the proximal end of the insertion tube. For other applications, the working or suction channel may be omitted.

In order to be able to manoeuvre the endoscope inside the body cavity, the distal end of the endoscope may comprise a bending section with increased flexibility, e.g. an articulated tip part assembly allowing the operator to bend this section. Typically, this is done by tensioning or slacking steering wires also running along the inside of the elongated insertion tube from the articulated tip part assembly to a control mechanism of the handle.

A general desire in the field of endoscopy is to electrically insulate the insertion tube, and thus especially the tip part assembly, from the outside, so as to mitigate the risk of an insulation breakdown and a resulting excessive leakage current.

Another general desire in the field of endoscopy is to provide a tip part assembly which is liquid-sealed, so as to mitigate liquid ingress into the tip part assembly, and specifically into any electrical or optical components of the tip part assembly.

For some types of endoscopes, such as urethroscope, there is a desire to provide the tip part assembly of the endoscope with a smaller diameter or cross sectional extent, especially where the tip part assembly is to be inserted into narrower body cavities. In very narrow body cavities, even a reduction of 1 mm or less in the cross-sectional extent of a tip part assembly can have a noticeable effect on the comfort of the patient and may even make it possible to reach body areas not otherwise accessible. Providing a small size of the tip part assembly can especially be a challenge in cases where the endoscope comprises both a camera and a working channel extending through the tip part assembly since the camera and working channel are positioned one above the other within the tip part assembly, which takes up space in a radial direction of the tip part assembly.

In some prior art tip part assemblies, a camera assembly with a flexible printed circuit (FPC) and a camera module are included. The FPC is connected to a connection surface proximally of or at a rear side of the camera module. The camera module comprises an image sensor and a lens stack positioned distally or in front of the image sensor. The image sensor may extend in a plane parallel to a plane, in which the printed circuit extends. When a working channel is included as well, the camera module is positioned above the working channel within a housing with a typically circular-cylindrical circumferential outer surface. The FPC lies side-by-side with and is connected to the connection surface of the camera module. From this connection, the FPC extends upwardly, i.e. away from the working channel, into a fold towards a proximal end of the housing. Further towards or beyond a proximal end of the housing where there is room proximally of or behind the connection surface of the camera module, the FPC includes electrical components for the camera module and potentially any LEDs.

It is therefore desirable to provide a tip part assembly with a small outer diameter for an endoscope, such as a urethroscope, having electrically insulating properties and with a mechanically stable printed circuit.

On this background it is desirable to provide an improved tip part assembly for an endoscope, which at least mitigates some of the abovementioned drawbacks.

### Summary

A first aspect of this disclosure relates to a tip part assembly for an endoscope, comprising:
a circuit board comprising a first major surface and a second major surface opposite the first major surface, wherein a first electrical connection pad is positioned on the first major surface, and a second electrical connection pad is positioned on the second major surface, and
a camera module comprising a connection surface with at least a first and a second connection point for electrical communication of the camera module with the circuit board, the first and second connection points being spaced apart from each other,
wherein a solidified electrical conductor filler material directly electrically connects the first connection point of the connection surface and the first electrical connection pad of the circuit board, and a solidified electrical conductor filler material directly electrically connects the second connection point of the connection surface and the second electrical connection pad of the circuit board.

It has been realized that in the above mentioned prior art tip part assemblies including a camera module and a printed circuit, a flexible printed circuit (FPC) is used to provide an electrical connection between the camera module and other parts of the endoscope. The connection surface of the camera module typically extends in a height direction, orthogonal or substantially orthogonal to a longitudinal and/or proximal-distal direction of the tip part assembly and/or endoscope, respectively. The FPC is therefore typically provided with a portion arranged at or adjacent to and connected to the camera module connection surface, a portion extending in the longitudinal and/or proximal-distal direction, and a fold interconnecting the two portions. So, instead of folding a FPC in a top volume of the housing as in the prior art, which means that a top volume of the housing above a top level of the camera module must provide room for the FPC fold, according to the present disclosure, a circuit board, such as a printed circuit board, is connected on two major sides thereof to the camera module. According to the present disclosure, the PCB may extend in the longitudinal and/or proximal-distal direction substantially without or without extending into the top volume. This means that the mentioned top volume of the housing is not necessary and can be dispensed with or be reduced. This again means that the extent of the housing in a height direction can be reduced. This is due to the height direction typically being decisive for the necessary diameter or cross-sectional extent of the housing since there is more room at the lateral side of the camera module and a working channel, where this is provided. Therefore, the diameter or the cross-sectional extent of the housing and thus the tip part assembly can therefore be reduced. This reduction may be from about 0.1 and up to 1 mm or more. Hereby, a patient's comfort during an endoscopy procedure may be improved, and the tip part assembly may even provide access to body areas not otherwise accessible. It has been shown that an outer diameter of the circumferential wall of the tip part assembly of less than 3 mm or even smaller may be achieved.

Another advantage may be that the PCB may be easier and/or cheaper to manufacture than a FPC, which in turn may allow for a lower manufacturing cost of the tip part assembly according to the present disclosure.

A further advantage may be that the PCB may ease an assembly of the tip part assembly, when a housing is provided, as the PCB when directly electrically connected onto the camera module may be used to guide and/or arrange the camera module.

In such a tip part assembly, a risk of electrical conduction from the electronics inside a housing, such as the PCB, and through the housing to the patient may be mitigated in use, as the PCB may be positioned with at a distance from the housing. The material thickness necessary for the housing material to provide sufficient electrically insulating properties, which may impact the tip part assembly cross-section or diameter, may be reduced to be generally of a size suitable for certain types of endoscopes, such as urethroscopes.

An outer diameter of a circumscribed circle in a cross-section of the tip part assembly may be reduced compared to a tip part assembly with an upwardly extending FPC and fold. Thus, the inner and outer cross-section and/or diameter of a housing, where such is provided, may be reduced, in turn allowing for a small tip part assembly.

The term "endoscope" may be defined as a device suitable for examination of natural and/or artificial body openings, e.g. for exploration of a lung cavity. Additionally, or alternatively, the term "endoscope" may be defined as a medical device.

In this specification, a proximal-distal direction may be defined as an axis extending along the parts of the insertion tube of the endoscope. Adhering to the definition of the terms distal and proximal, i.e. proximal being the end closest to the operator and distal being the end remote from the operator. The proximal-distal direction is not necessarily straight, for instance, if the insertion tube is bent, then the proximal-distal direction follows the curvature of the insertion tube. The proximal-distal direction may for instance be a centre line of the insertion tube.

The tip part assembly may have a proximal end for being connected to other parts of the endoscope and a distal end positioned oppositely from the proximal end for forming a distal end of the endoscope. A longitudinal direction may extend from the distal end towards the proximal end of the tip part assembly. A height direction may extend transversely to the longitudinal direction. A lateral direction may extend transversely to the longitudinal and height directions. Angles between these three directions may be right.

A distal end of the tip part assembly may form a distal end of the endoscope.

Throughout this specification, the term camera assembly may be defined as a sub-assembly of the tip part assembly. The camera assembly may comprise the camera module and the circuit board.

The circuit board may be a printed circuit board (PCB). Alternatively, the circuit board may be a flexible circuit board. Throughout this specification, the term circuit board and PCB may be used interchangeably.

The solidified electrical conductor filler material may be solidified solder, such as solder paste, and/or solder tin potentially comprising one or more of tin, selen, rosin, and/or glycol-based materials, may be an electrically conducting or electrically conductor glue or welding material. The solidified electrical conductor filler material may have a resistivity of less than 0.1 Ω·m.

The term "directly electrically connected" can potentially mean one or more of "soldered to", "welded to", "glued to", and/or "adhered to". The term "directly electrically connected" may mean that substantially nothing but the solidified electrical conductor filler material electrically connects the electrical connection pad and the connection point.

The term "connection point" as used herein may alternatively be denoted "connection" and is not intended to be limited to being points in a mathematical sense, but may rather extend over an area of some size.

One or more connection points may be provided as a connection pin and may include a solder, glue, and/or welding material. Alternatively, or additionally, one or more connection points may be provided as pads, such as solder pads, which may have an electrically conducting material on a surface thereof, or may be provided as solder balls. The connection point may, alternatively or additionally stem from a solder ball which was melted during manufacture. Where a plurality of connection points is provided as solder balls, the connection points may form ball grid array (BGA).

The connection surface connection points of the camera module may be provided as connection pins including a solder material and protruding from the connection surface. The connection surface connection pins may have a substantially circular or circular cross-section when seen in a direction perpendicular to the connection surface. Solder material may be applied and may be arranged in a substantially hemisphere shape or hemisphere shape on a connection surface connection point and/or connection pin.

In some embodiments, a cross-section of the connection surface connection points may have a length or diameter in the range from 150 µm - 300 µm, such as 230 µm.

Each of the first, second, and/or any additional electrical connection pad may be a solder pad, a welding pad, i.e. a pad configured to provide an electrical connection when receiving a welding material, or a gluing pad, i.e. a pad configured to provide an electrical connection when receiving a glue material. The first, second, and/or any additional electrical connection pads may be of the same type of pads or may be of different types of pads. At least part of the solidified electrical conductor filler material may be applied to the pad before manufacturing. The solidified electrical conductor filler material may be liquefied during manufacturing. Where the first, second, and/or any additional electrical connection pads are provided as solder pads, the solder pads may be provided with solder paste on a surface thereof.

In some embodiments, the first, second, and/or any additional electrical connection pad may have a rectangular or substantially rectangular cross-section having a first side length in the range from 0.2-1.5 mm, and a second side length in the range from 0.5 mm to 3 mm. The first, second, and/or any additional electrical connection pads may have a larger surface area than a cross-sectional area of the connection surface connection points.

The connection between respective connection points and/or electrical connection pads may be an electrical connection. An electrical connection may comprise electrical communication, e.g. by means of varying voltages, electric potentials and/or currents being applied to and/or running through, respectively, the one or more connection points. Alternatively, or in combination therewith, the electrical connection may comprise supplying a supply voltage, such as a V+ and V- connection, to the camera module.

The circuit board may be in electrical communication with additional electrical/electronic components or elements and may at least partly redirect or forward communication to/from the additional electrical/electronic components or elements from/to the camera module. Additional electrical/electronic components or elements may comprise one or more of passive components, such as capacitors, resistors, and/or inductors, semiconductors, such as integrated circuits (ICs), diodes, and/or transistors, cables or wires, and/or flexible flat cables (FFCs).

PCBs are well-known electronic items that can be manufactured by technologies such as laminating and etching. The PCB may, throughout this specification, comprise two or more copper layers and one or more layers of insulating materials, such as layers of a FR-4 (flame retardant) composite material. The PCB may be a two-layer or multi-layer PCB. In some embodiments, other electrically conductive materials may be used instead of or in combination with copper in conducting layers of the PCB.

The PCB may be rigid or non-flexible PCB. The PCB may have a thickness, i.e. from the first major surface to the second major surface, of less than 1.6 mm, and/or in the range from 0.1 - 1.6 mm, such as in the range from 0.1 - 0.8 mm, such as in the range from 0.1 - 0.5 mm. The two or more copper layers may have the same or a different thickness, such as a thickness of 0.05 mm or less, such as 0.035 mm. The PCB may further comprise solder masks, such as top and/or bottom solder masks, silkscreens, and/or pre-preg layers.

A first copper layer of the PCB may extend in parallel with or constitute the first major surface, and a second copper layer of the PCB may extend in parallel with or constitute the second major surface. The pads may be arranged adjacent to or abutting an end surface, such as a distal end surface, of the PCB. The distal end surface may abut and/or face the connection surface of the camera module. In some embodiments, the major surfaces of the PCB are formed by two outermost layers of copper, i.e. two layers of copper facing the exterior of the PCB.

In some embodiments, a solder mask and/or a silk screen may be applied on one or more of the copper layers of the PCB, such as on a surface thereof facing away from a layer of insulating material of the PCB. Where a solder mask and/or silk screen is applied, the surface on which this/these is/are applied may be defined as a major surface.

The camera module may have a housing, in which image sensor and lens stack may be arranged. Outer surfaces of the camera module or a camera module housing of the camera module may be substantially box-shaped and/or parallelepipedal. The camera housing may house at least a part of the lens stack and/or a part of the image sensor.

The lens stack may be positioned distally of or in front of the image sensor, may include two or more lenses and may include a proximal lens and a distal lens. The camera module may further comprise a lens barrel which may hold and encase the lens stack. The lens stack may be stacked and/or the lens barrel may extend in a longitudinal direction. The connection surface may be positioned proximally of or behind the image sensor. The connection surface may face in a proximal direction. The lens stack or the lens barrel may have a longitudinally extending centre line, which may be, or may be coinciding with, a centre line of the camera module.

The at least one lens, potentially the plurality of lenses, may be of one or more types chosen from the group consisting of: concave, convex, plano-concave, plano-convex, bi-convex, bi-concave.

The tip part assembly may further comprise a working channel. The working channel may be substantially tubular and/or have a circumferentially extending, potentially substantially cylindrical or circular cylindrical, outer wall enclosing a working channel spacing. The working channel may have an inner diameter of 0.8 to 2 mm or 1 to 1.6 mm or 1 to 1.4 mm. A wall thickness of a circumferential wall of the working channel may be 0.1 to 0.5 mm.

The working channel may comprise a chamfered portion, which may face at least a part of the camera assembly. The chamfered portion may, additionally or alternatively, be provided as a canted-off portion, a bevelled portion, or the like. The chamfered portion may be abutting at least part of the camera assembly or may be positioned with a distance to the camera assembly.

The chamfered portion may be part of a circumferential wall of the working channel, where a such is provided, or may be formed in one piece with the wall. The wall thickness of the chamfered portion of the circumferential wall may thus be smaller than along at least one other portion of the circumferential wall.

The working channel may allow liquid to be removed from a body cavity and/or allow insertion of surgical instruments or the like into the body cavity. The working channel may be provided as a channel extending from a proximal end of an endoscope to a distal end of the endoscope to guide a tool and/or to provide suction. A connector and/or a connecting portion may be provided at the proximal end of the endoscope to allow insertion of a tool into the working channel and/or to allow suction to be applied to the working channel. In some embodiments, the working channel comprises a built-in or integrated tool at or in the distal tip part assembly. Such a tool may be suitable for grabbing, taking, and/or holding elements in a part of a patient, in which the endoscope tip part assembly is arranged during use.

In some embodiments, the working channel may similarly have a longitudinally extending centre line, which may extend from a proximal end to a distal end of the working channel. The centre line of the working channel may furthermore be parallel to a centre line of the of the camera module and/or a centre line of the insertion tube.

The camera module and a working channel may be positioned side-by-side or bottom-to-top. A top surface of the camera module may be positioned adjacent to and may be abutting or in contact with an inner top surface of the circumferential wall of a housing of the tip part assembly. See further below regarding such a housing. A bottom surface of the working channel may be positioned adjacent to and may be abutting or in contact with an inner bottom surface of the circumferential wall of the housing.

If the tip part assembly includes a working channel, the camera module may be positioned above the working channel within the housing so that a top surface of the camera module is positioned adjacent to the wall and a bottom surface of the camera module is positioned adjacent to the working channel. The camera module may be positioned above the working channel in the height direction.

The tip part assembly may comprise one or more light emitting diodes (LEDs). The LED(s) may be arranged at the distal end of the tip part assembly or abutting one or more light guides (see description below) arranged at the distal end of the tip part assembly, and may have a connection surface extending substantially in parallel or in parallel to the connection surface of the camera module. The LED(s) may be arranged beside the camera module in a lateral direction. The tip part assembly may comprise circuitry, such as a flexible printed circuit (FPC), for electrically connecting one or more LED(s) to other parts of the endoscope, such as the printed circuit. Alternatively, or additionally, the LED(s) may be mounted on the printed circuit.

The tip part assembly may comprise one or more LED holders. The LED holder(s) may comprise at least one first portion extending in or substantially in the longitudinal direction. The LED holder first portion may abut an LED so as to support the LED. The LED holder may, where e.g. two or more LEDs are provided, comprise a second portion, which may abut and support the other LED(s). The LED holder first and second portions may be interconnected by at least one interconnecting portion of the LED holder. The interconnecting portion may be arranged proximally, i.e. further towards the proximal end of the tip part assembly, of the LED(s). The LED holder may be made from a material comprising or consisting of a polymer, such as polyethylene (PE), a crylonitrile butadiene styrene (ABS), or the like, and may be manufactured by means of extrusion, moulding, and/or 3D-printing. The LED holder may be manufactured in one piece or may comprise several pieces adjoined, e.g. by means of an adhesive.

The tip part assembly may also include one or more light guides for guiding light from respective LED(s) to e.g. a front or distal end surface or end wall of the tip part assembly and/or a housing thereof. One or more light guides may be in one piece, potentially moulded in one piece with, a housing of the tip part assembly. The light guide(s) may extend from the distal end of the tip part assembly to a respective LED or a respective set of LEDs. In some embodiments, the light guides are made from a transparent material. The light guide(s) may be moulded and/or may comprise a portion abutting the camera module and/or be arranged in front of the lens stack.

The LEDs may comprise a light emitting surface. The light emitting surface(s) may emit light in the proximal-distal direction. The light emitting surface(s) may be positioned in abutment with a housing, where this is provided, or in abutment with one or more light guides.

Alternatively, or additionally, the tip part assembly may comprise one or more light fibres for conveying light from an external or proximal light source to the distal end of the endoscope.

The camera assembly may comprise a mounting frame. The mounting frame may support and/or secure one or more in the group consisting of: the printed circuit board, one or more light sources, such as LED(s), potentially provided at the distal end of the tip part assembly, the lens barrel, and the image sensor.

An exterior flexible sleeve may be provided to extend around at least part of the tip part assembly, potentially of a housing thereof and/or around at least part of an insertion tube of the endoscope

A maximum extent in the longitudinal direction from the distal end of the tip part assembly to a proximal end of the housing or circuit board is in some embodiments 12, 10 or 8 mm.

The tip part assembly may further comprise a bending section which may have a distal end segment which may be connected to a housing of the tip part assembly.

This may allow for the tip part assembly to be manoeuvred inside the body cavity. Thereby, various places inside the body cavity may be inspected by means of the camera assembly, and/or the working tool or suction may, by means of the working channel, be applied at various places inside the body cavity.

The bending section may comprise a number of hingedly interconnected segments including a distal end segment, a proximal end segment, and a plurality of intermediate segments positioned between the proximal end segment and the distal end segment. At least one hinge member may interconnect adjacent segments with each other. The bending section may be a section allowing the tip part assembly to bend relative to an insertion tube, potentially so as to allow an operator to manipulate the tip part assembly while inserted into a body cavity of a patient. The bending section may be moulded in one piece or may be constituted by a plurality of moulded pieces.

In some embodiments, the housing may be connected to the distal end segment of the bending section. The housing may be connected to the distal end segment at the proximal end of the housing. The working channel may extend into and/or through the distal end segment and/or the bending section.

The camera module may have a distal end and a proximal end opposite the distal end. The connection surface of the camera module may be located at the proximal end of the camera module.

A housing of the tip part assembly may extend in a longitudinal direction between a distal end and a proximal end of the housing. The connection surface may extend transversely to the longitudinal direction, such as in a height direction and/or in a lateral direction.

The distal end of the camera module may face and/or form at least part of the distal end of the tip part assembly.

The first and second connection points of the connection surface and/or any further connection points may each individually protrude from the connection surface towards the proximal end of the tip part assembly.

Each connection surface connection point may be provided separately and/or at a distance from and/or not be in physical contact with the other connection points.

Each connection surface connection point may be provided as one connection pin and/or as a solder ball that protrudes from the connection surface. Each connection surface connection point may protrude towards a proximal end of the tip part assembly. Each connection surface connection point may protrude in a longitudinal direction of the tip part assembly.

The camera module may comprise a lens stack, at least a portion of the lens stack being located at the distal end of the camera module.

The housing may be an outer or exterior housing which may be exterior with respect to elements housed or enclosed therein, such as the camera module, the circuit board, wires, electrical components, LEDs, and the like. Additionally or alternatively, the housing may be comprised in a distal end segment of a bending section of the tip part assembly.

An outer maximum extent in a lateral direction of the tip part assembly may be less than 3.3 mm.

This outer maximum extent may a maximum outer diameter of the tip part assembly and/or may be a maximum cross sectional extent. The outer maximum extent may be less than 3.2, 3.1, 3.0. 2.9, 2.8, 2.7, 2.6, or 2.5 mm.

The circuit board may be arranged so that the first connection point of the connection surface is positioned adjacent to the first electrical connection pad. Alternatively, or additionally, the second connection point of the connection surface may be positioned adjacent to the second electrical connection pad.

At least one electrical/electronic component or element may be arranged on the circuit board. The electrical/electronic component or element may be in electrical connection with the first or second electrical connection pads. The electrical connection may be an electrical connection as described above with respect to the connection points.

The first and second electrical connection pads of the circuit board may be arranged at a distance from each other corresponding to or substantially corresponding to a distance between the first and second connection points of the connection surface.

The first and second electrical connection pads may be arranged at a distance from each other equal to or substantially equal to a distance between the first and second connection points of the connection surface. Alternatively, or additionally, the first and second connection points may be arranged at a distance from each other corresponding to or substantially corresponding to a distance between the first and second electrical connection pads. The first and second connection points may be arranged at a distance from each other equal to or substantially equal to a distance between the first and second electrical connection pads.

Additionally or alternatively, the first and second connection points may be positioned at a distance from each other corresponding to or substantially corresponding to a thickness of the circuit board or a distance between the first and second major surfaces of the circuit board. the first and second connection points may be positioned at a distance from each other equal to or substantially equal a to thickness of the circuit board or a distance between the first and second major surfaces of the circuit board.

The camera module may comprise a third connection point and a fourth connection point. The first and third connection points may be arranged in a first row. The second and fourth connection points may be arranged in a second row. A third electrical connection pad may be positioned on the first major surface, and a fourth electrical connection pad may be positioned on the second major surface. A solidified electrical conductor filler material may directly electrically connect the third connection point of the connection surface and the third electrical connection pad of the circuit board. A solidified electrical conductor filler material may directly electrically connect the fourth connection point of the connection surface and the fourth electrical connection pad of the circuit board.

In the present embodiment, the third or the fourth sets of connection points and electrical connection pads may be left out.

The two rows may be arranged at a distance from each other substantially equal to a distance between the first and second electrical connection pads. Additionally or alternatively, the two rows may be positioned at a distance from each other substantially corresponding to thickness of the circuit board or a distance between the first and second major surfaces of the circuit board.

The two rows may be positioned at a distance from each other.

In some embodiments, the four connection surface connection points are each provided as solder balls having a substantially circular or circular cross-section. The cross-section of the connection surface connection points may have a diameter in the range from 150 µm - 300 µm, such as 230 µm.

The four connection surface connection points may be arranged so that each connection point is positioned in a respective corner of a substantially rectangular shape. A first and a second side of the rectangular shape, respectively, may be defined between the geometric centres of a connection surface connection point and two other connection surface connection points, so that an angle between the first and second sides of the rectangle is right or substantially right. The first side may have a length, i.e. a distance between the geometric centres of the two connection points defining this side, in the range of 350 µm - 450 µm , such as 400 µm. The second side may have a length, i.e. a distance between the geometric centres of the two connection points defining this side, in the range of 400 µm - 550 µm, such as 470 µm.

The thickness of the circuit board may correspond to or substantially correspond to, such as be equal to or be less than, the length of the first and/or the second side of the rectangular shape.

The circuit board may be positioned to extend in between the first and second connection points of the connection surface.

Alternatively, or additionally, the circuit board may extend in between the first and second rows of connection points.

When the tip part assembly comprises a housing as mentioned above, the distal end of the circuit board may extend substantially transversely to the longitudinal direction.

Both of the first and second major surfaces of the circuit board may include at least one electronic component.

The circuit board may have a distal end and a proximal end. The distal end of the circuit board may be positioned between the first and second connection points of the connection surface.

Both of the first and second major surfaces of the circuit board may similarly include connection points, which may be printed on the circuit board, and which connect the electrical component on the first major surface to the electrical connection pad on the first major surface, and which connect the electrical component on the second major surface to the electrical connection pad on the second major surface. As such, the circuit board may be a circuit board printed on two surfaces.

A respective electronic component may be mounted on the first and/or the second side, respectively. The electronic component may be mounted by means of soldering, such as infrared soldering, through-hole mounting, surface mounting technology (SMT), ACF/ACA-bonding, or the like.

An electronic component may alternatively or additionally be an electrical element and may comprise one or more from the group of: passive components, semiconductors, integrated circuits, electromechanical components, plugs, connectors, sockets, cables, wires, and/or flexible flat cables (FFCs).

In some embodiments, the at least one electronic component comprises a surface mount device (SMD) capacitor, and four wires. The capacitor may be a ceramic or film capacitor and may be in a 0402- or 0201-sized package (approximately 0.4 x 0.2 mm, or 0.25 mm x 0.125 mm, respectively) and may be soldered onto the first major surface of the circuit board. Two wires may be soldered onto the first major surface of the circuit board, potentially proximally of the first and potentially third electrical connection pads and/or proximally of the capacitor, and two wires may be soldered onto the second major surface of the circuit board, potentially proximally of the second and potentially fourth electrical connection pads.

The circuit board may extend substantially in a plane. Alternatively or additionally, the circuit board may extend in a plane.

The circuit board first and second major surfaces may be positioned substantially in planes.

The circuit board may generally have a rectangular, such as a square, shape and/or may be free of folds.

One or more or all of the connection points of the connection surface may be provided as solder balls that have been soldered onto the respective electrical connection pads. The electrical connection pads may be provided as solder pads.

Upon soldering, the solder material of the solder balls as well as solder paste, where this is provided on the solder pads, may be melted and adjoined, or additional solder material, such as tin solder, may be provided for interconnecting the solder balls and solder pads.

The tip part assembly may further comprise a housing extending in a longitudinal direction between a distal end and a proximal end of the housing. The housing may comprise a circumferential wall enclosing a spacing. At least a portion of the camera module and a portion of the circuit board may be arranged in the spacing. The connection surface may extend transversely to the longitudinal direction.

The camera module and/or the circuit board may be at least partly or fully shielded electrically and/or from liquid from the body of a patient when the endoscope is in use. This may, in turn, mitigate a risk of failure due to electrical short-circuiting as well as a risk of electrically shocking a patient on which the endoscope is used.

Furthermore, an advantage of the tip part assemblies according to this disclosure may be that the need for material of the housing to provide electrical insulation may be reduced by the circuit board. By connecting the circuit board, by the first and second major surfaces thereof, to the camera module, the circuit board may be positioned further from the housing wall than an FPC with a fold arranged in the top volume, thereby using the dielectric properties of the air or of a material arranged between the circuit board and the housing wall, such as a potting material where such is provided in the housing.

The housing may comprise an end wall arranged at a distal end of the housing and/or forming the distal end of the housing. The end wall may be formed in one piece with other parts of the housing. The end wall may comprise an opening to the exterior of the housing for a working channel and/or may comprise a window towards the exterior of the housing for the camera module.

In some embodiments a potting material, such as an adhesive, may be provided in the housing. The potting material may fill out any or parts of a spacing between the components, e.g. the working channel, the camera module, and/or the circuit board, or the sections thereof arranged inside the housing.

The housing may be tubular and/or cylindrical or substantially cylindrical and/or circular cylindrical or substantially circular cylindrical. The working channel or a part thereof may be in one piece with the housing and/or may be provided as a separate part. The working channel may comprise a first portion potentially provided in one piece with the housing, and a second portion interconnected with the first portion. The working channel second portion may be provided as a flexible tube and may be interconnected to the first portion by means of an adhesive.

The housing may provide electrical insulation and/or water tightness around the circuit board and electrical connections within the housing and may form a mould or container for adhesive and/or potting material poured or injected into the housing. The housing may ensure that a minimum insulation thickness is present on one or more sides or all outer surfaces of the tip part assembly. If an adhesive or a potting material is present in the housing, this may provide greater robustness, mechanical stability and/or rigidity of the tip part assembly, and/or better attachment/fixation of components within the housing. This may be advantageous since wires or cables may be pulled during operation of the endoscope, pulling also in the circuit board, especially during bending of a bending section of the tip part assembly.

Moulding of the housing may occur as a two-component moulding in which two different materials are moulded in the same mould. For example, an end wall arranged at and/or forming a distal end of the housing may be moulded in a first material, which may be transparent, and the circumferential wall may be moulded in a second, different material, which may be non-transparent and may include higher adhesive compatibility with an adhesive or potting material in the housing. Alternatively, the circumferential wall can be manufactured separately from the end wall. For example, the two walls can be moulded separately, or the end wall can be moulded, and the circumferential wall extruded. In this case, the circumferential wall and the end wall can be adhered to each other by means of an adhesive. Alternatively, the circumferential wall may be moulded in the same material as the end wall, and/or the housing may be moulded in one piece in one material, such as a transparent material.

The housing circumferential wall may be a tubular wall enclosing a volume, the volume being a spacing within the housing. This spacing may also be defined by a distal end wall of the housing. An opening for the working channel may be provided in the end wall. The camera module and potentially other components of the tip part assembly may be at least partly housed within the spacing and potentially attached, potentially by means of adhesive, to the housing. The spacing may be at least partly filled with a hardened adhesive, the hardened adhesive being provided separately from the housing. Alternatively, the housing may take the form of a hardened adhesive in which the camera module and potentially other components may be embedded.

A lateral portion of the volume of the housing may be defined laterally from the camera module and the working channel, the lateral volume extending between the proximal end and the distal end of the housing.

A second aspect of this disclosure relates to a method of manufacture of a tip part assembly for an endoscope, the method comprising the steps of:
a) providing a circuit board of the tip part assembly comprising a first major surface and a second major surface opposite the first major surface, wherein a first electrical connection pad is positioned on the first major surface, and a second electrical connection pad is positioned on the second major surface,
b) providing a camera module of the tip part assembly comprising a connection surface with at least a first and a second connection point for electrical communication of the camera module with the circuit board, the first and second connection points being spaced apart from each other,
c) directly connecting the first connection point of the connection surface to the first electrical connection pad of the circuit board by a liquefied electrical conductor filler material, and
d) subsequent to step c) allowing the liquefied electrical conductor filler material to solidify so that the first electrical connection pad and the first connection point are directly electrically connected by solidified electrical conductor filler material,
e) directly connecting the second connection point of the connection surface to the second electrical connection pad of the circuit board by a liquefied electrical conductor filler material,
f) subsequent to step e) allowing the liquefied electrical conductor filler material to solidify so that the second electrical connection pad and the second connection point are directly electrically connected by solidified electrical conductor filler material.

The steps of the method according to the second aspect may, except where stated otherwise, be performed in any order, not necessarily in sequence. The steps may be carried out in the sequence a), b), c), d), e), f) or may alternatively be carried out in the sequence a), b), e), f) c), d), the sequence b), a), c), d), e), f) or the sequence b), a), e), f) c), d). Steps a) and b) may be performed simultaneously.

The method may provide identical or similar advantages to the tip part assembly according to the first aspect of this disclosure. Embodiments of the tip part assembly of this method may be the same as described with respect to the tip part assembly according to the first aspect of this disclosure.

Step c) may comprise directly connecting the first connection point of the connection surface to the first electrical connection pad of the circuit board by a liquefied first electrical conductor filler material. Step d) may further comprise allowing the liquefied first electrical conductor filler material to solidify. Step e) may comprise directly connecting the second connection point of the connection surface to the second electrical connection pad of the circuit board by a liquefied second electrical conductor filler material. Step f) may further comprise allowing the liquefied second electrical conductor filler material to solidify. The first and second electrical conductor filler materials may be made from the same or different materials.The method may furthermore comprise a step of, before steps c) and d), arranging the circuit board between the first and second connection surface connection point.

The method may further comprise the steps of:
g) providing a housing of the tip part assembly, the housing extending in a longitudinal direction between a distal end and a proximal end of the housing, said housing comprising a wall enclosing a spacing,
h) subsequent to steps c) and e), arranging at least a portion of the camera module and at least a portion of the circuit board in the spacing of the housing.
Step g) may be performed before, after, or simultaneously with one or more of steps a), b), c), d), e) and/or f).

Preferably the entire camera module is arranged in the housing.

The housing may be a housing as described above with respect to the first aspect of this disclosure, i.e. the housing may be tubular and/or cylindrical or substantially cylindrical and/or circular cylindrical or substantially circular cylindrical and/or may comprise a tubular and/or comprise a circumferential wall and/or an end wall. Where the housing comprises a camera holding section, the camera module may be arranged therein in step f).

By directly connecting the circuit board to the camera module before arranging this in the housing, the circuit board may be used to guide and/or hold the camera module in the housing and/or in the camera holding section. Where one or more wires and/or cables are connected to the circuit board, these may similarly be used to guide the camera module and circuit board.

A third aspect of this disclosure relates to a tip part assembly for an endoscope, the tip part assembly being manufactured according to the second aspect of the disclosure.

This tip part assembly may provide identical or similar advantages to the tip part assembly according to the first aspect of this disclosure. Embodiments of the tip part assembly of this method may be the same as described with respect to the tip part assembly according to the first aspect of the disclosure.

A fourth aspect of the present disclosure relates to an endoscope comprising a tip part assembly according to the first aspect of the disclosure or a tip part assembly according to the third aspect of the disclosure.

The endoscope may comprise a control element. The control element may be configured to allow an operator to control a tip part assembly of the insertion tube by at least one steering wire. The control element may allow bending the tip part assembly in at least one direction, potentially in two directions, the two directions potentially being opposite. The control element may be accommodated in an operating handle. The control element may include a lever allowing an operator to control the control element. The lever may extend outwardly from the control element, potentially through an operating handle. The control element may be in the form of a roller or a roller disc.

The endoscope may comprise an operating handle. The operating handle may be suitable for allowing an operator to grip and to operate the endoscope, potentially with one hand. The operating handle may comprise a handle housing arranged at a proximal end of the insertion tube. The handle housing may accommodate the control element.

The insertion tube and/or a distal end thereof and/or the tip part assembly thereof may be suitable for insertion into a body cavity, potentially a kidney, through a body opening, potentially a urinary passage or a urethra. The body may be a natural and/or artificial body, potentially a human body. The insertion tube may extend from the operating handle towards a distal end of the endoscope.

Additionally or alternatively, the endoscope may form part of a system for visually inspecting inaccessible places such as human body cavities, the system further comprising a monitor. The endoscope may be connectable to the monitor, and the monitor may allow an operator to view an image captured by the camera assembly of the endoscope.

A person skilled in the art will appreciate that any one or more of the above aspects of this disclosure and embodiments thereof may be combined with any one or more of the other aspects of the disclosure and embodiments thereof.

### Brief description of the drawings

The tip part assemblies and methods will now be described in greater detail based on non-limiting exemplary embodiments and with reference to the drawings, on which:
FIG. 1a shows a perspective view of an endoscope in which a tip part assembly according to the present disclosure is implemented,
FIG. 1b shows a perspective view of a monitor to which the endoscope of FIG. 1a is connected,
FIG. 2a shows a top-down view of a first camera assembly of the tip part assembly of FIGs. 1a and 1b,
FIG. 2b shows a side view of the first camera assembly of the tip part assembly of FIGs. 1a and 1b,
FIG. 2c shows a top-downview of a second, alternative camera assembly of an embodiment of a tip part assembly according to the present disclosure,
FIG. 3a shows a perspective view of a circuit board of the first camera assembly of the tip part assembly of FIGs. 1a and 1b,
FIG. 3b shows a perspective view of a camera module of the first camera assembly of a tip part assembly of FIGs. 1a and 1b,
FIG. 3c shows a perspective view of the first camera assembly of the tip part assembly of FIGs. 1a and 1b, seen from below in a height direction,
FIG. 4a shows a distal end plane view of a first embodiment of a tip part assembly of FIGs. 1a and 1b,
FIG. 4b shows a cross-sectional view of the tip part assembly of FIGs. 1a and 1b,
FIG. 4c shows a second cross-sectional view of the tip part assembly of FIGs. 1a and 1b, taken orthogonally to the cross-sectional view of FIG. 4b along the line A-A of FIG. 4a,
FIG. 5a shows a perspective view of the tip part assembly of FIGs. 1a and 1b,
FIG. 5b shows a perspective view of a second, alternative embodiment of a tip part assembly according to the present disclosure,
FIG. 6 shows a plane view of a bending section of the endoscope of FIG. 1a and FIG. 1b.

Similar reference numerals are used for similar elements across the various embodiments and figures described herein.

### Detailed description

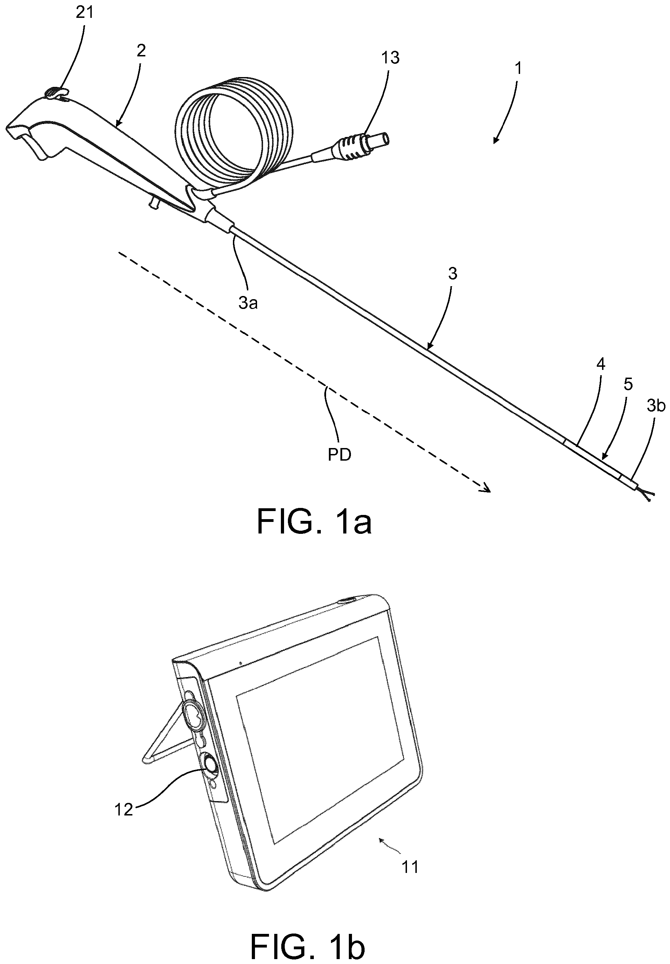

Referring first to FIG. 1a, an endoscope 1 is shown. The endoscope is disposable, and not intended to be cleaned and reused. The endoscope 1 comprises an elongated insertion tube 3. At the proximal end 3a of the insertion tube 3 an operating handle 2 is arranged. The operating handle 2 has a control lever 21 for manoeuvring a tip part assembly 5 at the distal end 3b of the insertion tube 3 by means of a steering wire. A camera assembly 6 is positioned in the tip part assembly 5 and is configured to transmit an image signal through a monitor cable 13 of the endoscope 1 to a monitor 11.

In Fig. 1b, a monitor 11 is shown. The monitor 11 may allow an operator to view an image captured by the camera assembly 6 of the endoscope 1. The monitor 11 comprises a cable socket 12 to which a monitor cable 13 of the endoscope 1 can be connected to establish a signal communication between the camera assembly 6 of the endoscope 1 and the monitor 11.

The proximal-distal direction PD is an axis extending along the parts of the insertion tube 3 of the endoscope 1.

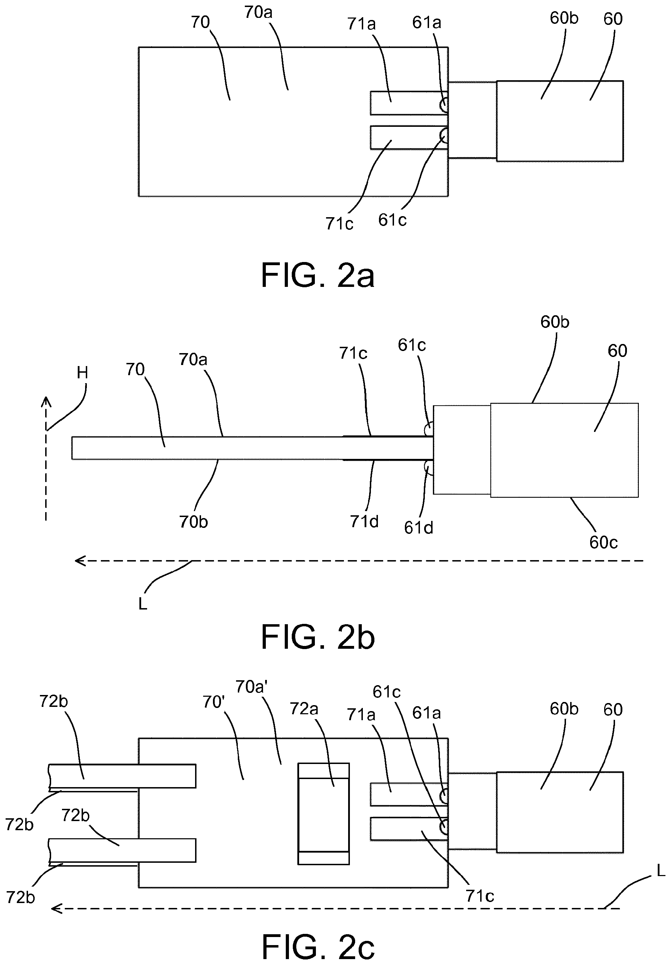

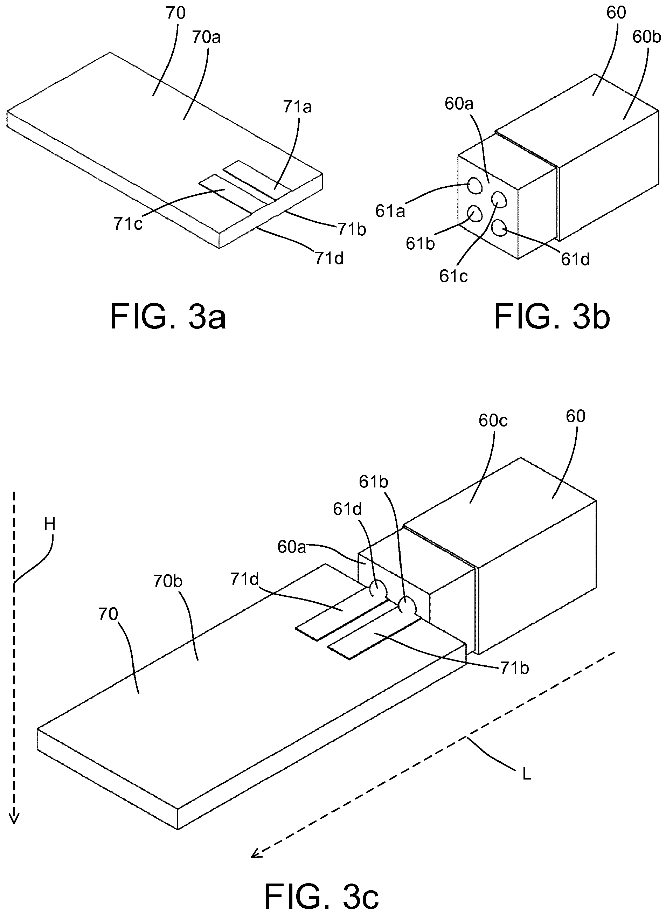

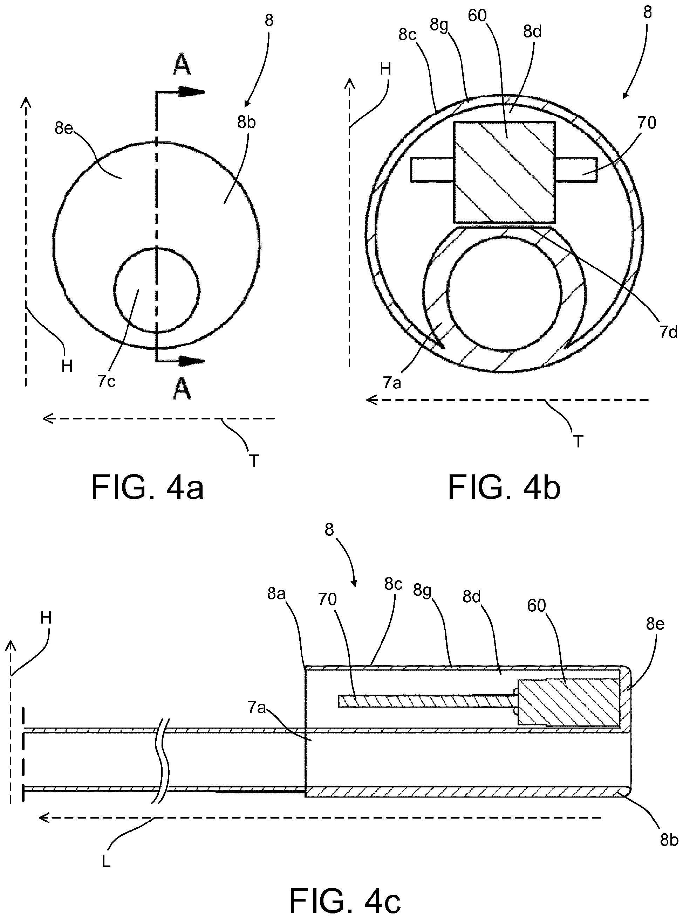

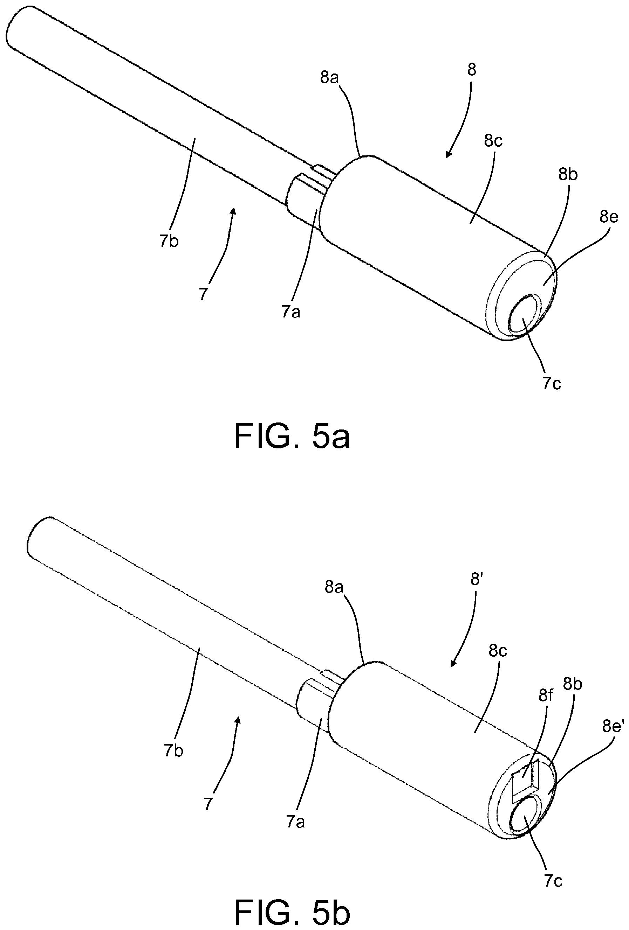

Referring to FIGs. 2a and 2b, a camera assembly 6 is shown. FIGs. 3a-3c show a camera module 60 and a printed circuit board, PCB, 70 of the camera assembly 6 of FIGs. 2a and 2b. The PCB 70 has a first 70a and second major surface 70b opposite the first major surface 70a. FIGs. 4a-4c and FIGs. 5a and 5b show a tip part assembly comprising the camera assembly 6 of FIGs. 2a and 2b.

Referring to FIG. 2c, another embodiment of a camera assembly 6' is shown comprising a camera module 60 similar to that of the camera assembly 6 shown in FIGs. 2a and 2b and a printed circuit board, PCB, 70'. Electronic components are mounted on the PCB 70', i.e. a SMD capacitor 72a and two of four wires 72b are mounted on a first major surface 70a' of the PCB 70'. Two other of the four wires 72b are mounted on a second major surface (not shown) opposite the first major surface 70a' of the PCB 70'. The second major surface of the PCB 70' is similar to the second major surface 70b of the PCB 70 of the first embodiment of the camera assembly 6.

The tip part assembly 5 has a proximal end for being connected to other parts of the endoscope 1 and a distal end positioned oppositely from the proximal end for forming a distal end of the endoscope 1. A longitudinal direction L extends from the distal end towards the proximal end of the tip part assembly 5. A height direction H extends transversely to the longitudinal direction. A lateral direction T extends transversely to the longitudinal L and height directions H. Angles between these three directions are right.

The distal end of the tip part assembly 5 forms a distal end of the endoscope 1.

A first solder pad 71a is positioned on the first major surface 70a, 70a', and a second solder pad 71b is positioned on the second major surface 70b. The camera module 60 comprises a connection surface 60a with at least a first 61a and a second connection point 61b for electrical communication of the camera module 60 with the circuit board 70, 70'. The first 61a and second connection points 61b are spaced apart from each other.

The first connection point 61a of the connection surface 60a is soldered to the first solder pad 71a of the circuit board 70, 70', and the second connection point 61b of the connection surface 60a is soldered to the second solder pad 71b of the circuit board 70, 70'.

The camera module 60 has a distal end and a proximal end opposite the distal end. The connection surface 60a of the camera module 60 is located at the proximal end of the camera module 60.

The tip part assembly 5 comprises a housing 8, 8' extending in the longitudinal direction L between a distal end 8b and a proximal end 8a of the housing. The housing distal end 8b here forms the distal end of the tip part assembly 5, and the housing proximal end 8a forms the proximal end of the tip part assembly 5. The tip part assembly 5 further comprises a working channel 7.

The camera module 60 comprises a third connection point 61c and a fourth connection point 61d. The first 61a and third connection points 61c are arranged in a first row. The second 61b and fourth connection points 61d are arranged in a second row. A third solder pad 71c is positioned on the first major surface 70a, 70a', and a fourth solder pad 71d is positioned on the second major surface 71b. The third connection point 61c of the connection surface 60a is soldered to the third solder pad 71c of the circuit board 70, 70', and the fourth connection point 61d of the connection surface 60a is soldered to the fourth solder pad 71d of the circuit board 70. 70'.

The two rows are arranged at a distance from each other substantially equal to a distance between the first 71a and second solder pads 71b. The two rows are positioned at a distance from each other substantially corresponding to thickness of the circuit board 70, 70', i.e. a distance between the first 70a, 70a' and second major surfaces 70b of the circuit board 70, 70'.

The four connection surface connection points 61a-61d are arranged so that each connection point is positioned in a respective corner of a substantially rectangular shape as seen e.g. on FIG. 3b. A first and a second side of the rectangular shape, respectively, is defined between the geometric centres of a connection surface connection point 61a and two other connection surface connection points 61b, 61c, so that an angle between the first and second sides of the rectangle is right or substantially right. The first side has a length, i.e. a distance between the geometric centres of the two connection points 61a, 61b defining this side, of approximately 400 µm. The second side has a length, i.e. a distance between the geometric centres of the two connection points 61a, 61c defining this side, of approximately 470 µm.

Each of the connection points 61a-61d are provided as connection pins and include a solder material, i.e. as solder balls, and together form a ball grid array (BGA). The connection surface connection points 61a-61d have a circular cross-section when seen in a direction perpendicular to the connection surface 60a. The solder material is applied and arranged in a hemisphere shape on each of the connection surface connection points 61a-61d.

A cross-section of the connection surface connection points 61a-61d has a diameter of approximately 230 µm.

The first 71a, second 71b, third 71c and fourth solder pads 71d have a rectangular cross-section having a first side length in the range from 0.2-1.5 mm, and a second side length in the range from 0.5 mm to 3 mm. The solder pads 71a-71d are each provided with solder paste on a surface thereof. The solder pads each have a larger surface area than a cross-sectional area of the connection surface connection points 61a-61d.

The PCB 70, 70' comprises two copper layers, on which the solder pads 71a-71d are arranged and one layers of FR-4 (flame retardant) composite material in between the two copper layers. The two copper layers form the first 71a and second major surfaces 71b.

The PCB 70, 70' is a rigid PCB. The PCB has a thickness, i.e. seen in the height direction and from the first major surface to the second major surface, in the range from 0.2-0.4 mm. The two or more copper layers have the same thickness, such as a thickness of 0.035 mm.

A first copper layer of the PCB 70, 70' constitutes the first major surface 70a, 70a', and a second copper layer of the PCB 70, 70' constitutes the second major surface 70b. The pads 71a-71d are arranged abutting a distal end surface of the PCB 70, 70'. The distal end surface of the PCB 70, 70' faces the connection surface 60a of the camera module 60. The distal end of the circuit board 70, 70' extends in the lateral direction T, i.e. substantially transversely to the longitudinal direction L.

The camera module 60 has a housing, in which an image sensor (not shown) and a lens stack (not shown) are arranged and housed. Outer surfaces of the camera module 60 are box-shaped.

The camera module 60 is positioned above the working channel 7 seen in the height direction H. A top surface 60b of the camera module 60 is positioned with a distance to an inner top surface of the circumferential wall 8g of a housing 8 of the tip part assembly 5.

The distal end of the camera module 60 faces part of the distal end 8b of the tip part assembly 5, i.e. a proximal surface of the end wall 8e.

Each connection surface connection point 61a-61d is provided separately at a distance from and not in physical contact with the other connection points 61-61d. Each connection surface connection protrudes towards a proximal end of the tip part assembly, i.e. the proximal end 8a of the housing. The connection surface connection points 61a-61d protrude in the longitudinal direction L of the tip part assembly 5.

The circuit board 70, 70' is arranged so that the first connection point 61a of the connection surface 60a is positioned adjacent to the first solder pad 71a. The second connection point 61b of the connection surface 60a is positioned adjacent to the second solder pad 71b.

The electronic components 72a, 72b are each electrically connected to at least one of the solder pads 71a-71d.

The first 71a and second solder pads 71b of the circuit board 70 are arranged at a distance from each other equal to a distance between the first 61a and second connection points 61b of the connection surface 60a. Similarly, the third 71c and fourth solder pads 71d of the circuit board 70 are arranged at a distance from each other equal to a distance between the third 61c and fourth connection points 61d of the connection surface 60a.

The circuit board 70 is positioned to extend in between the first 61a and second connection points 61b of the connection surface 60 as well as in between the first and second rows of connection points 61a-61d.

The SMD capacitor 72a is mounted by means of surface mounting technology (SMT). The four wires 72b are each mounted to the PCB 70, 70' by means of soldering.

The SMD capacitor 72a is a ceramic capacitor in a 0201-sized package. Two wires 72b are soldered onto the first major surface 70a, 70a' of the PCB 70, 70', proximally of the first 71 a and third solder pads 71c and proximally of the SMD capacitor 72a. The other two wires 72b are soldered onto the second major surface 72b of the PCB 70, 70', proximally of the second 71b and fourth solder pads 71d.

The circuit board 70, 70' extends in a plane. The circuit board first 70a, 70a' and second major surfaces 70b are positioned in planes. The circuit board 70, 70' generally has a rectangular shape and is free of folds.

All of the connection points 61a-61d of the connection surface 60a are soldered onto the respective solder pads 71a-71d.

Turning to FIGs. 4a-5b, the tip part assembly comprises a housing 8, 8' having a circumferentially extending outer surface 8c for facing the environment. The outer surface 8c encloses a volume and extends in the longitudinal direction L between the distal end 8b and proximal end 8b of the housing 8, 8'. The working channel 7 is partly housed in the housing 8, 8' and comprises an opening 7c in a distal surface, i.e. the end wall 8e, 8e', of the housing 8, 8'. The camera module 60 is housed in the housing 8, 8'. The circuit board 70 is arranged in the housing 8, 8'. A top volume 8d of the housing 8, 8' is defined above the camera module 60 in the height direction H and extending along the housing 8, 8' in the longitudinal direction L.

The housing comprises an end wall 8e, 8e' forming the distal end 8b of the housing 8, 8'. The end wall 8e, 8e' comprises an opening to the exterior of the housing for the working channel 7. The end wall opening is provided in one piece with the working channel opening 7c. The end wall 8e further comprises a window 8f, i.e. an opening, towards the exterior of the housing 8, 8' for the camera module 60.

The housing 8, 8' is moulded in one piece, and the circumferential wall 8g is moulded in the same material as the end wall 8e, 8e', i.e. in a transparent material.

The housing 8, 8' is tubular and circular cylindrical. The working channel 7 comprises a first portion 7a potentially provided in one piece with the housing 8, 8', and a second portion 7b interconnected with the first portion 7a. The working channel second portion 7b is provided as a flexible tube and is interconnected to the first portion 7a by means of an adhesive.

A maximum extent in the longitudinal direction L from the distal end 8b to the proximal end 8a of the housing 8, 8' is 10 mm.

The connection surface 60a of the camera module 60 extends in the height direction H as well as in the lateral direction T, orthogonal to the longitudinal L and/or the proximal-distal direction PD of the tip part assembly 5 and/or endoscope 1, respectively. So, instead of folding a FPC in a top volume 8d of the housing 8, 8' as in the prior art, which means that a top volume 8d of the housing 8, 8' above a top level, i.e. a top surface 60b of the camera module 60 must provide room for a FPC fold, according to the present disclosure, the circuit board 70, 70' is connected on two major sides 70a, 70a', 70b thereof to the camera module 60. The PCB 70, 70' extends in the longitudinal L without extending into the top volume 8d of the housing 8, 8'. This means that the mentioned top volume 8d of the housing 8, 8' is reduced compared to the prior art. This again means that the extent of the housing in the height direction H can be reduced. An outer diameter of the circumferential wall 8g of the housing 8, 8' is 2.9 mm. Thus, an outer maximum extent in the lateral direction T of the tip part assembly 5 is 2.9 mm.

The tip part assembly 5 comprises a working channel 7. The working channel 7 is tubular and has a circumferentially extending, circular cylindrical, outer wall enclosing a working channel spacing. The working channel 7 has an inner diameter of 1.2 mm. A wall thickness of a circumferential wall of the working channel 7 is 0.3 mm.

The working channel 7 comprises a chamfered portion 7d, which faces the camera assembly 6, 6'. The chamfered portion 7d faces a bottom surface 60c of the camera module 60. The chamfered portion 7d is positioned with a distance to the camera assembly 6, 6'.

The chamfered portion 7d is formed in one piece with the circumferential wall of the working channel 7.

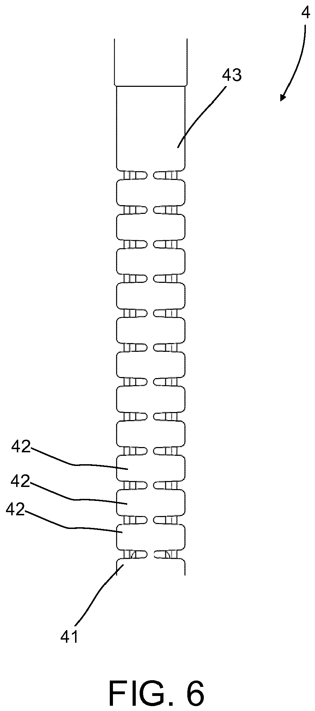

Turning to FIG. 6, a bending section 4 is provided. The bending section 4 comprises a number of hingedly connected segments including a distal end segment 41, a proximal end segment 43, and a plurality of intermediate segments 42 positioned between the distal end segment 41 and the proximal end segment 43. The distal end segment 41 is adapted for being connected and/or attached to a housing of a tip part assembly, such as the housing 8 of FIGs. 4a-4c and 5a or the housing 8' of FIG. 5b, at a proximal end of the housing 8, 8'.

### List of references

The following is a list of reference numerals used throughout this specification.
- 1: endoscope
- 11: monitor
- 12: cable socket
- 13: monitor cable
- 2: handle
- 21: control lever
- 3: insertion tube
- 3a: proximal end
- 3b: distal end
- 4: bending section
- 41: distal end segment
- 42: intermediate segment
- 43: proximal end segment
- 5: tip part assembly
- 6: camera assembly
- 6': camera assembly
- 60: camera module
- 60a: camera module connecting surface
- 60b: camera module top surface
- 60c: camera module bottom surface
- 61a: first connection point
- 61b: second connection point
- 61c: third connection point
- 61d: fourth connection point
- 7: working channel
- 7a: first working channel portion
- 7b: second working channel portion
- 7c: working channel distal opening
- 7d: working channel chamfered portion
- 70: printed circuit board (PCB)
- 70': printed circuit board (PCB)
- 70a: PCB first major surface
- 70a': PCB first major surface
- 70b: PCB second major surface
- 71a: first solder pad
- 71b: second solder pad
- 71c: third solder pad
- 71d: fourth solder pad
- 72a: SMD capacitor
- 72b: wires
- 8: housing
- 8': housing
- 8a: housing proximal end
- 8b: housing distal end
- 8c: outer surface
- 8d: lateral spacing portion
- 8e: housing end wall
- 8e': housing end wall
- 8f: window
- L: longitudinal direction
- PD: proximal-distal direction
- S: symmetry plane
- TD: top-down direction

## Claims

1. A tip part assembly for an endoscope, comprising:
a circuit board comprising a first major surface and a second major surface opposite the first major surface, wherein a first electrical connection pad is positioned on the first major surface, and a second electrical connection pad is positioned on the second major surface, and
a camera module comprising a connection surface with at least a first and a second connection point for electrical communication of the camera module with the circuit board, the first and second connection points being spaced apart from each other,
wherein a solidified electrical conductor filler material directly electrically connects the first connection point of the connection surface and the first electrical connection pad of the circuit board, and a solidified electrical conductor filler material directly electrically connects the second connection point of the connection surface and the second electrical connection pad of the circuit board.

2. A tip part assembly according to claim 1, wherein the tip part assembly has a proximal end for being connected to other parts of the endoscope and a distal end positioned oppositely from the proximal end for forming a distal end of the endoscope, the camera module having a distal end and a proximal end opposite the distal end, wherein the connection surface of the camera module is located at the proximal end of the camera module.

3. A tip part assembly according to claim 1 or 2, wherein the circuit board is arranged so that the first connection point of the connection surface is positioned adjacent to the first electrical connection pad and/or the second connection point of the connection surface is positioned adjacent to the second electrical connection pad.

4. A tip part assembly according to any one of the preceding claims, wherein the first and second electrical connection pads of the circuit board are arranged at a distance from each other substantially corresponding to a distance between the first and second connection points of the connection surface.

5. A tip part assembly according to any one of the preceding claims, wherein the camera module further comprises a third connection point and a fourth connection point, the first and third connection points being arranged in a first row, the second and fourth connection points being arranged in a second row, wherein a third electrical connection pad is positioned on the first major surface, and a fourth electrical connection pad is positioned on the second major surface, and wherein a solidified electrical conductor filler material directly electrically connects the third connection point of the connection surface and the third electrical connection pad of the circuit board, and a solidified electrical conductor filler material directly electrically connects the fourth connection point of the connection surface and the fourth electrical connection pad of the circuit board.

6. A tip part assembly according to any one of the preceding claims, wherein the circuit board is positioned to extend in between the first and second connection points of the connection surface.

7. A tip part assembly according to any one of the preceding claims, wherein the tip part assembly has a proximal end for being connected to other parts of the endoscope and a distal end positioned oppositely from the proximal end for forming a distal end of the endoscope, wherein the circuit board has a distal end and a proximal end, and the distal end of the circuit board is positioned between the first and second connection points of the connection surface.

8. A tip part assembly according to any one of the preceding claims, wherein both of the first and second major surfaces of the circuit board include at least one electronic component.

9. A tip part assembly according to any one of the preceding claims, wherein the circuit board extends substantially in a plane.

10. A tip part assembly according to any one of the preceding claims, wherein the circuit board is a printed circuit board.

11. A tip part assembly according to any one of the preceding claims, wherein one or more or all of the connection points of the connection surface are provided as solder balls that have been soldered onto the respective electrical connection pads, the electrical connection pads being provided as solder pads.

12. A tip part assembly according to any one of the preceding claims further comprising a housing extending in a longitudinal direction between a distal end and a proximal end of the housing, said housing comprising a circumferential wall enclosing a spacing, wherein at least a portion of the camera module and a portion of the circuit board are arranged in the spacing, and wherein the connection surface extends transversely to the longitudinal direction- outer dimensions, circumferential, tubular wall and spacing.

13. A method of manufacture of a tip part assembly for an endoscope, the method comprising the steps of:
a) providing a circuit board of the tip part assembly comprising a first major surface and a second major surface opposite the first major surface, wherein a first electrical connection pad is positioned on the first major surface, and a second electrical connection pad is positioned on the second major surface,
b) providing a camera module of the tip part assembly comprising a connection surface with at least a first and a second connection point for electrical communication of the camera module with the circuit board, the first and second connection points being spaced apart from each other,
c) directly connecting the first connection point of the connection surface to the first electrical connection pad of the circuit board by a liquefied electrical conductor filler material, and
d) subsequent to step c) allowing the liquefied electrical conductor filler material to solidify so that the first electrical connection pad and the first connection point are directly electrically connected by solidified electrical conductor filler material,
e) directly connecting the second connection point of the connection surface to the second electrical connection pad of the circuit board by a liquefied electrical conductor filler material,
f) subsequent to step e) allowing the liquefied electrical conductor filler material to solidify so that the second electrical connection pad and the second connection point are directly electrically connected by solidified electrical conductor filler material.

14. A method according to claim 13 further comprising the steps of:
g) providing a housing of the tip part assembly, the housing extending in a longitudinal direction between a distal end and a proximal end of the housing, said housing comprising a wall enclosing a spacing,
h) subsequent to steps c) and e), arranging at least a portion of the camera module and at least a portion of the circuit board in the spacing of the housing.

15. A tip part assembly manufactured according to any one of claims 13-14.

16. An endoscope comprising a tip part assembly according to any one of claims 1-12 or according to claim 15.
